(19) Europäisches Patentamt
European Patent Office
Office européen des brevets

(11) Veröffentlichungsnummer: **0 287 084 B1**

(12) **EUROPÄISCHE PATENTSCHRIFT**

(45) Veröffentlichungstag der Patentschrift: **02.10.91**

(51) Int. Cl.$^5$: **C07C 33/20**, C07C 43/23, //C07C41/26,C07C29/136

(21) Anmeldenummer: **88105918.2**

(22) Anmeldetag: **14.04.88**

# (54) 2-Methyl- oder 2-Methoxyphenylgruppen enthaltende Alkohole und deren Verwendung als Duftstoffe.

(30) Priorität: **15.04.87 DE 3712873**

(43) Veröffentlichungstag der Anmeldung:
**19.10.88 Patentblatt 88/42**

(45) Bekanntmachung des Hinweises auf die Patenterteilung:
**02.10.91 Patentblatt 91/40**

(84) Benannte Vertragsstaaten:
**CH DE FR GB LI NL**

(56) Entgegenhaltungen:
**EP-A- 0 076 493**
**EP-A- 0 217 159**

(73) Patentinhaber: **Consortium für elektrochemische Industrie GmbH**
**Zielstattstrasse 20**
**W-8000 München 70(DE)**

(72) Erfinder: **Hafner, Walter, Dr.**
**Birkenalle 17**
**W-8196 Eurasburg(DE)**
Erfinder: **Friedrich, Wilhelm**
**Ernst-Platz-Strasse 34/0**
**W-8000 München 50(DE)**
Erfinder: **Gebauer, Helmut, Dr.**
**Schaffhauserstrasse 18/7**
**W-8000 München 71(DE)**
Erfinder: **Regiert, Marlies**
**Seitzstrasse 19**
**W-8000 München 22(DE)**

**EP 0 287 084 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Eichenmoosriechstoffe sind bekannt, jedoch sind dies zumeist Phenolderivate wie 3-Methyl-5-methoxyphenol mit den bekannten störenden phenolischen Eigenschaften wie Ätz- oder Reizwirkung und Verfärbungen.

Es ist ferner aus J. Indian Chem. Soc., Vol. L, September 1973 2-Methyl-3-(2-methylphenyl)-propan-1-ol bekannt, ohne daß in der genannten Veröffentlichung eine Verwendung angegeben wurde.

Aufgabe der Erfindung war das Auffinden von Duftstoffen mit Eichenmooscharakter, die hohe Stabilität aufweisen und keine störenden Eigenschaften wie Ätz-, Reizwirkung oder Verfärbungen zeigen.

Gegenstand der Erfindung sind Verbindungen der Formel

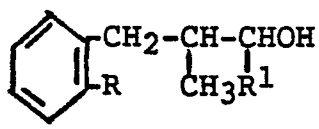

wobei R Methyl- oder Methoxyrest bedeutet, wenn $R^1$ ein Wasserstoffrest ist, und R Methylrest bedeutet, wenn $R^1$ ein Methylrest ist, und 2-Methyl-3-(2-methylphenyl)-propan-1-ol ausgeschlossen ist.

Ein Verfahren zur Herstellung von 2-Methyl-3-(2-methyl-phenyl)-propan-1-ol und 2-Methyl-3-(2-methoxyphenyl)propan-1-ol ist dadurch gekennzeichnet, daß

a) 2-Methylbenzaldehyd oder 2-Methoxybenzaldehyd mit Propionaldehyd in Gegenwart von Basen umgesetzt wird und

b) das Reaktionsprodukt gemäß a) hydriert wird.

Die als Ausgangsverbindungen verwendeten Benzaldehyde und Propionaldehyd sind bekannte Substanzen.

Die Umsetzung gemäß a) erfolgt vorzugsweise mit Alkalimetallhydroxyden insbesondere Natriumhydroxyd oder Kaliumhydroxyd bei Temperaturen von vorzugsweise -5° C bis 80° C in polaren Lösungsmitteln wie Alkoholen beispielsweise Methanol oder Ethanol. Das Reaktionsprodukt gemäß a) wird dann mit Reduktionskatalysatoren wie Palladium auf Aktivkohle oder Raney-Nickel und Wasserstoff hydriert und schließlich durch fraktionierende Destillation in Aldehyd bzw. Alkohol separiert.

Ein weiteres Verfahren zur Herstellung von 2-Methyl-3-(2-methylphenyl)-propan-1-ol oder 2-Methyl-3-(2-methoxyphenyl)-propan-1-ol ist dadurch gekennzeichnet, daß

a1)2-Methylbenzylchlorid oder 2-Methoxybenzylchlorid mit Methylmalonsäurediethylester umgesetzt wird,

b1) das Reaktionsprodukt gemäß a1) nach erfolgter Verseifung thermisch decarboxyliert wird und

c1)das Reaktionsprodukt gemäß b1) hydriert wird.

Die verwendeten Benzylchloride und Methylmalonsäurediethylester sind bekannte Verbindungen. Die Umsetzung gemäß a1) erfolgt vorzugsweise bei Temperaturen von 70 bis 120° C in Gegenwart einer Base wie Natrium- oder Kaliumcarbonat. Nach erfolgter Verseifung beispielsweise mit Basen wie Natriumhydroxyd oder Kaliumhydroxyd wird vorzugsweise bei 120 bis 150° C thermisch decarboxyliert. Die erhaltene Säure wird gegebenenfalls nach erfolgter Veresterung mit Alkoholen wie Methanol oder Ethanol mit Hydrierungsmitteln wie Lithiumaluminiumhydrid oder Wasserstoff in Gegenwart eines Katalysators umgesetzt.

Ein Verfahren zur Herstellung von 3-Methyl-4-(2-methylphenyl)-butan-2-ol ist dadurch gekennzeichnet, daß 2-Methylbenzylchlorid mit Methylethylketon in einem organischen/alkalischen Zweiphasensystem in Gegenwart eines Phasentransferkatalysators umgesetzt wird.

2-Methylbenzylchlorid und Methylethylketon sind bekannte Verbindungen. Methylethylketon wird vorzugsweise im Überschuß eingesetzt. Das organisch/alkalische Zweiphasensystem wird gebildet aus den Reaktionskomponenten, sowie gegebenenfalls einem organischen mit Wasser nicht mischbaren inerten Lösungsmittel und einer 5-50%-igen wäßrigen Lösung von Alkalimetallhydroxyd oder in fester Form vorliegendem Alkalimetallhydroxyd oder Alkalimetallcarbonat. Beispiele für Alkalimetallhydroxyde sind Natriumhydroxyd oder Kaliumhydroxyd. Als Phasentransferkatalysatoren werden beispielsweise Kronenether, quaternäre Ammonium- oder Phosphoniumsalze in Mengen von 0,5-5 Mol-% bezogen auf Benzylchlorid eingesetzt. Das Reaktionsprodukt dieser Umsetzung wird dann in an sich bekannter Weise mit Reduktionsmitteln wie Natriumborhydrid, Lithiumaluminiumhydrid oder Wasserstoff und Katalysator zum 3-Methyl-4-(2-methylphenyl)-butan-2-ol reduziert.

Bezüglich weiterer Einzelheiten wird auf die Beispiele verwiesen.

Gegenstand der Erfindung ist weiterhin die Verwendung der erfindungsgemäßen Verbindungen und von 2-Methyl-3-(2-methylphenyl)-propan-1-ol als Duftstoffe.

Die erfindungsgemäßen Duftstoffe zeigen Eichenmooscharakter und hohe chemische und physikalische Stabilität. Aufgrund ihrer Beständigkeit haben sie ein sehr weites Anwendungsgebiet. Neben dem Einsatz in der Parfümerie eignen sie sich auch zur Beduftung von Wasch- und Reinigungsmitteln, Textilien, Kunststofferzeugnissen u.a.

Beispiel 1
2-Methyl-3-(2-methoxyphenyl)-propan-1-ol

Unter Argon-Spülung wurden 6,5g KOH in 200ml Ethanol (95%) gelöst und auf 4° C abgekühlt. Dann wurden 98g 2-Methoxybenzaldehyd dazugegeben und im Verlauf von 4,5 Stunden 45g Propionaldehyd zugetropft. Die Reaktionstemperatur wurde bei 8-9° C gehalten. Nach 15min Nachreaktion wurde mit Eis, Wasser und Ether versetzt und die organische Phase abgetrennt. Sie wurde viermal mit je 200ml Wasser ausgeschüttelt und über eine 19cm Füllkörperkolonne mit Glaswendeln destilliert. Nach Abtrennung von Lösungsmittel und Vorlauf wurde von 86-92° C bei 0,07mbar eine Fraktion (88g) mit einem Gehalt von 94% 2-(2-Methoxybenzyliden)-propionaldehyd erhalten. 87g dieser Substanz, 150ml Ethanol und 10g Raney-Nickel wurden in einen Schüttelautoklaven unter 100bar Wasserstoffdruck 14 Stunden auf 130° C erhitzt. Nach Entfernung von Katalysator und Lösungsmittel wurde über die oben erwähnte Kolonne destilliert. Es wurden bei 98° C/0,13mbar 70g 2-Methyl-3-(2-methoxyphenyl)-propan-1-ol erhalten.

Geruchsnote: erdig-phenolisch, vergleichbar mit der Charakteristik von Wurzelkörper und Eichenmoos

Beispiel 2
2-Methyl-3-(2-methylphenyl)-propan-1-ol

220g 2-Methylbenzylchlorid, 320g Methylmalonsäurediethylester, 320g Kaliumcarbonat, 13g Kaliumjodid und 10g 18-Krone-6 wurden in 11 Toluol 12 Stunden bei 90° C gerührt.

Die erkaltete Mischung wurde mit 800g Eis in Wasser verrührt, die wäßrige Schicht abgetrennt und einmal ausgeethert. Die vereinigten organischen Schichten wurden dreimal mit je 400ml Wasser ausgeschüttelt. Dann wurden, zum Teil bei vermindertem Druck, Lösungsmittel und nicht umgesetzter Methylmalonsäurediethylester abdestilliert. Der Rückstand (413g) wurde mit 540g 25- %iger NaOH unter Zusatz von einer Spatelspitze Tetradecyltrimethylammoniumbromid verseift (14 Stunden Rückfluß). Dabei wurde freigesetztes Ethanol abdestilliert und etwas Wasser zugegeben. Dann wurde mit konz. Salzsäure angesäuert, die organische Phase mit Xylol aufgenommen und zur Decarboxylierung langsam einige Stunden bis zum Rückfluß erhitzt. Anschließend wurde das Xylol wieder abdestilliert und die zurückgebliebene 2-Methyl-3-(2-methylphenyl)-propionsäure mit Äthanol und Cyclohexan azeotrop verestert. Der Ester wurde über eine 20cm Füllkörperkolonne destilliert (220g, Siedepunkt 68-71° C bei 0,1-0,2mbar).
In einem 21-Vierhalskolben wurden 1,21 trockenes Tetrahydrofuran vorgelegt, unter Argon 38g Lithiumaluminiumhydrid dazugegeben und unter Eiskühlung eine Mischung von 200g obigen Esters mit 200ml Ether zugetropft. Nach einer Nachreaktion von 2 Stunden bei Raumtemperatur wurde der Ansatz auf Wasser gegossen, mit Salzsäure angesäuert und die Schichten getrennt. Die wäßrige Phase wurde zweimal ausgeethert, die vereinigten organischen Phasen mit Sodalösung ausgerührt und destilliert (20cm Füllkörperkolonne mit Glaswendeln). Es wurden 148g 2-Methyl-3-(2-methylphenyl)-propan-1-ol mit einem Siedepunkt von 80° C bei 0,13mbar erhalten.
Geruchsnote: Eichenmoos und Vetiver

Beispiel 3
3-Methyl-4-(2-methylphenyl)-butan-2-ol

56g KOH, 56ml Wasser, 10g 18-Krone-6 und 5g KJ wurden mit 200ml Toluol unter Luftausschluß auf 75° C erwärmt. Unter kräftigem Rühren wurde in 30 Minuten eine Mischung von 100g 2-Methylbenzylchlorid und 100g Methylethylketon zugetropft und das Ganze 11 Stunden bei 85° C gerührt. Dann wurden die Phasen getrennt, die organische Schicht mit Wasser ausgeschüttelt und über eine kurze Füllkörperkolonne destilliert. Nach Abtrennung von Lösungsmittel und Vorlauf wurden bei 54-55° C/0,07mbar 29g 3-(2-Methylbenzyl)-butanon erhalten. 25g davon wurden mit 3g $NaBH_4$ in 100ml Ethanol zunächst 2 Stunden bei 25° C, dann weitere 2 Stunden bei Rückflußtemperatur gerührt. Nach dem Entfernen von Reduktionsmittel und Lösungsmittel wurde das Reaktionsprodukt über eine kleine Vigreux-Kolonne destilliert. Von 64-67° C/0,07mbar wurden 15,5g 3-Methyl-4-(2-methylphenyl)-butan-2-ol erhalten.
Geruchsnote: Eichenmoos, mit süß-puderiger Komponente

## Patentansprüche

1. Verbindungen der Formel

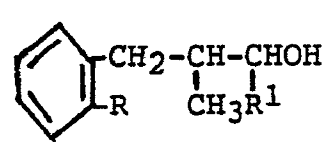

wobei R Methoxy bedeutet, wenn $R^1$ Wasserstoff ist, und R Methyl bedeutet, wenn $R^1$ Methyl ist.

2. Verwendung der Verbindungen nach Anspruch 1 und von 2-Methyl-3-(2-methylphenyl)-propan-1-ol als Duftstoffe.

## Claims

1. Compounds of the formula

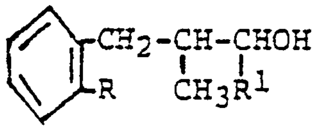

where R denotes methoxy when $R^1$ is hydrogen, and R denotes methyl when $R^1$ is methyl.

2. Use of the compounds according to Claim 1 and of 2-methyl-3-(2-methylphenyl)-propan-1-ol as fragrances.

**Revendications**

1. Composés de formule

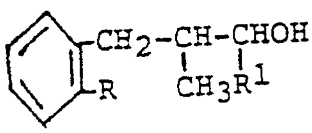

dans laquelle R est le radical méthoxy quand $R^1$ est un hydrogène et R est le radical méthyle quand $R^1$ est le radical méthyle.

2. Utilisation des composés selon la revendication 1 et du méthyl-2 (méthyl-2 phényl)-3 propanol-1 en tant que parfums.